(19) Europäisches Patentamt

European Patent Office

Office européen des brevets

(11) EP 0 826 696 B1

(12) EUROPÄISCHE PATENTSCHRIFT

(45) Veröffentlichungstag und Bekanntmachung des Hinweises auf die Patenterteilung:
29.05.2002 Patentblatt 2002/22

(51) Int Cl.7: C07K 16/46, A61K 39/395

(21) Anmeldenummer: 97115190.7

(22) Anmeldetag: 02.09.1997

(54) **Verwendung bi-und trispezifischer Antikörper zur Induktion einer Tumorimmunität**

Use of bi-and trispecific antibodies for inducing tumor immunity

Utilisation des anticorps bi- et trispécifiques pour induire une immunité antitumorale

(84) Benannte Vertragsstaaten:
AT BE CH DE DK ES FR GB IT LI NL SE

(30) Priorität: 03.09.1996 DE 19635743
26.11.1996 DE 19648976
13.03.1997 DE 19710497

(43) Veröffentlichungstag der Anmeldung:
04.03.1998 Patentblatt 1998/10

(73) Patentinhaber: GSF-Forschungszentrum für Umwelt und Gesundheit GmbH
85764 Oberschleissheim (DE)

(72) Erfinder:
• Lindhofer, Horst, Dr.
82194 Gröbenzell (DE)
• Kolb, Hans-Jochem, Prof.-Dr.
80804 München (DE)
• Thierfelder, Stefan, Prof.-Dr.
82223 Eichenau (DE)

(74) Vertreter: Behnisch, Werner, Dr.
Reinhard-Skuhra-Weise & Partner,
Patentanwälte,
Postfach 44 01 51
80750 München (DE)

(56) Entgegenhaltungen:
EP-A- 0 637 593

• LINDHOFER H. ET AL.: "Bispecific Antibodies Target Operationally Tumor-Specific Antigens in Two Leukemia Relapse Models" BLOOD, Bd. 88, 15.Dezember 1996, Seiten 4651-4658, XP000616201

• LINDHOFER,H. ET AL: "Bispecific antibodies effectively purge cancer cells from peripheral blood stem cell collections without affecting colony forming units " 26TH ANNUAL MEETING OF THE INTERNATIONAL SOCIETY FOR EXPERIMENTAL HEMATOLOGY, CANNES, FRANCE, AUGUST 24-28, 1997, Bd. 25, Nr. 8, 1997, EXPERIMENTAL HEMATOLOGY (CHARLOTTESVILLE), Seite 879 XP002048523

• WEINER G.J. ET AL.: "The role of Tcell activation in anti-CD3 X antitumor bispecific antibody therapy" JOURNAL OF IMMUNOLOGY, Bd. 152, 1994, Seiten 2385-2392, XP002048527

• WEINER, G.J. AND DE GAST G.C.: "Bispecific monoclonal antibody therapy of B-cell malignancy" LEUKEMIA AND LYMPHOMA, Bd. 16, 1995, Seiten 199-207, XP002048528

• WEINER L.M. ET AL.: "Clinical development of 2B1, a bispecific murine monoclonal antibody targeting c-erbB-2 and Fc-gamma-RIII" JOURNAL OF HEMATOTHERAPY, Bd. 4, 1995, Seiten 453-456, XP002048529

• SILLA L.M.R. ET AL.: "Potentiation of lysis of leukaemia cells by a bispecific antibody to CD33 and CD16 (Fc-gamma-RIII) expressed by human natural killer (NK) cells" BRITISH JOURNAL OF HAEMATOLOGY, Bd. 89, 1995, Seiten 712-718, XP002048530

• CHEN J. ET AL: "MONOCYTE-MEDIATED LYSIS OF ACUTE MYELOID LEUKEMIA CELLS IN THE PRESENCE OF THE BISPECIFIC ANTIBODY 251 X 22 (ANTI-CD33 X ANTI-CD64 )" CLINICAL CANCER RESEARCH, Bd. 1, Nr. 11, 1995, Seiten 1319-1325, XP000608204

• J. of Hematotherapy, Bd. 4, 1995, Seiten 433-437

• Cancer Immunol. Immunother., Bd. 40, 1995, Seiten: 390-396

Bemerkungen:

Die Akte enthält technische Angaben, die nach dem Eingang der Anmeldung eingereicht wurden und die nicht in dieser Patentschrift enthalten sind.

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft die Verwendung intakter bispezifischer und/oder trispezifischer Antikörper zur Induktion einer Tumorimmunität bei Mensch und Tier.

Maligne Erkrankungen des Menschen, beispielsweise Brustkrebs im fortgeschrittenen Stadium, haben trotz der Fortschritte der Chemo- und Radiotherapie in den letzten Jahren noch immer eine äußerst ungünstige Prognose. Eine Heilung dieser Erkrankungen ist nicht möglich. Es ist daher notwendig, neue Behandlungsstrategien zu entwikkeln. Große Hoffnungen werden dabei auf immuntherapeutische Ansätze gesetzt, mittles derer das Immunsystem des Patienten dazu gebracht werden soll, den Tumor abzustoßen. Es ist bekannt, daß auf Tumorzellen tumorassoziierte Antigene vorkommen und daß das Immunsystem prinzipiell durchaus in der Lage ist, diese zu erkennen und die malignen Zellen anzugreifen. Tumoren haben jedoch verschiedene Strategien entwickelt, die es ihnen erlauben, sich der Immunantwort zu entziehen. Dies gelingt ihnen z.B. durch ungenügende Präsentation von tumorassoziierten Antigenen und/oder durch unzureichende Aktivierung der in der Regel vorhandenen tumorspezifischen T-Zellen.

Bei etwa 43.000 Neuerkrankungen / Jahr steht Brustkrebs an der Spitze der Krebsstatistik für Frauen in Deutschland. Weniger als ein Drittel der Frauen mit Lymphknotenbefall bei Diagnose leben 10 Jahre ohne Rückfall.

Vor diesem Hintergrund wird seit einigen Jahren versucht, mit Hilfe der autologen Knochenmark- und Blutstammzell-Transplantation in Verbindung mit der Hoch-Dosis-Therapie Patientinnen mit ausgedehntem Lymphknotenbefall und Fernmetastasen eine Lebensverlängerung oder sogar Heilung zu ermöglichen. Trotz hoher Ansprechraten bei der Hoch-Dosis-Therapie ist eine Heilung im metastasierten Stadium selten.

Immuntherapeutische Ansätze beim Mamma-Karzinom beschränkten sich bisher auf Methoden zur unspezifischen Stimulation wie die Behandlung mit BCG oder Levamisol, sowie den Einsatz von LAK- und NK-Zellen mit IL-2. Eine Lebensverlängerung konnte mit bisher eingesetzten Formen von Immuntherapie nicht nachgewiesen werden, die Behandlung mit BCG war sogar eher nachteilig. Nachdem unspezifische Aktivierungen von Zellen auch bei anderen Tumorarten wenig Erfolg gehabt haben, sollte nun versucht werden, eine spezifische Immunreaktion in Gang zu bringen.

Für die Tumortherapie wurden beispielsweise T-Zell-redirigierende bispezifische Antikörper verwendet. Diese binden mit einem Bindungsarm an einen T-Zell-Rezeptorkomplex und mit dem zweiten Bindungsarm an ein tumorassoziiertes Antigen auf einer Tumorzelle. Durch die daraus resultierende Aktivierung der T-Zelle und der räumlichen Nähe der Tumorzelle wird letztere durch Apoptose-Induktion bzw. Cytokine wie TNF-$\alpha$ oder Perforin zerstört.

Es ist eine Aufgabe der vorliegenden Erfindung, ein neues Mittel bereitzustellen, um maligne Erkrankungen des Menschen zu therapieren.

Diese Aufgabe wird erfindungsgemäß durch die im Anspruch 1 näher gekennzeichneten Merkmale gelöst. Bevorzugte Ausgestaltungen der Erfindung ergeben sich aus den Unteransprüchen.

Die Erfindung offenbart somit die Verwendung eines intakten bispezifischen oder trispezifischen Antikörper mit den nachfolgenden Eigenschaften und Wirkungen:

(a) bindet über CD3 an eine T-Zelle und vermittelt ihr ein 1. Aktivierungssignal;

(b) bindet an tumorassoziierte Antigene auf einer Tumorzelle;

(c) bindet durch seinen Fc-Teil (bei bispezifischen Antikörpern) oder eine dritte Spezifität (bei trispezifischen Antikörpern) an den Fc-Rezeptor von Fc-Rezeptor positiven Zellen;

(d) aktiviert die Fc-Rezeptor positive Zelle durch seine Bindung an die Fc-Rezeptor positive Zelle, wodurch die Expression von Cytokinen und/oder von kostimulatorischen Antigenen initiiert oder erhöht wird;

(e) die kostimulatorischen Antigene und/oder Cytokine übertragen an die T-Zelle zumindest ein 2. Aktivierungssignal, das für eine physiologische Aktivierung der T-Zelle benötigt wird, wobei sich diese Aktivierung in der Hochregulation von Aktivierungsmarkern, der Zerstörung der Tumorzelle und/oder in einer Proliferation der T-Zelle zeigt; zur Herstellung eines Arzneimittels zur Induktion einer Tumorimmunität bei Mensch und Tier.

Die erfindungsgemäß verwendeten Antikörper sind bevorzugt in der Lage, die tumorspezifischen T-Zellen, die über den T-Zell-Rezeptor ein tumorspezifisches Peptid, welches über MHC Klasse I und/oder Klasse II auf Tumorzellen präsentiert wird, erkennen, nach Bindung durch den bispezifischen oder trispezifischen Antikörper, wie unter (e) beschrieben, zu aktivieren.

Die erfindungsgemäß verwendeten Antikörper sind weiterhin zur Reaktivierung von in Anergie befindlichen, tumorspezifischen T-Zellen befähigt. Weiterhin sind sie zur Induktion von tumorreaktiven Komplement-bindenden Antikörpern und damit zur Induktion einer humoralen Immunantwort in der Lage.

Die Bindung erfolgt über CD3 an die T-Zelle. Die Fc-Rezeptor positiven Zellen weisen zumindest einen Fcγ-Rezeptor I, II oder III auf.

Erfindungsgemäß verwendbare Antikörper sind zur Bindung an Monozyten, Makrophagen und/oder Dendriten als Fcγ-Rezeptor I positive Zellen befähigt.

Die erfindungsgemäß verwendbaren Antikörper bewirken, daß die Expression von CD40, CD80, CD86, ICAM-1 und/oder LFA-3 als kostimulatorische Antigene oder/und die Sekretion von Cytokinen durch die Fc-Rezeptor positive Zelle initiiert oder erhöht wird. Die Cytokine sind bevorzugt IL-1, IL-2, IL-4, IL-6, IL-8, IL-12 und/oder TNF-α.

Die Bindung an die T-Zelle erfolgt bevorzugt über den T-Zell-Rezeptor-Komplex der T-Zelle.

Der erfindungsgemäß verwendete bispezifische Antikörper ist ein anti-CD3 X anti-Tumor-assoziiertes Antigen-Antikörper.

Der erfindungsgemäß verwendete trispezifische Antikörper ist ein anti-CD3 X anti-Tumor-assoziiertes Antigen-Antikörper mit einem zusätzlichen anti-Fc-Rezeptor-Bindungsarm.

Bezugnehmend auf das Merkmal (a) wird das 1. Signal beispielsweise über den T-Zell-Rezeptor-Komplex der T-Zelle übertragen und kann damit, für sich allein betrachtet, zu einer unphysiologischen Aktivierung der T-Zelle führen. Die Zelle wird hierdurch anergisiert und kann auf T-Zell-Rezeptor vermittelte Stimuli nicht mehr angemessen reagieren. Durch die erfindungsgemäßen bispezifischen oder trispezifischen Antikörper wird zusätzlich gleichzeitig durch die kostimulatorischen Antigene auf der Fc-Rezeptor positiven Zelle an die T-Zelle ein 2. Aktivierungssignal übertragen, das zu einer physiologischen Aktivierung der T-Zelle und in der Folge entweder zu einer Zerstörung der Tumorzelle und/oder einer Proliferation der T-Zelle führt. Als weiteres Kriterium für die Aktivierung der T-Zelle kann die Hochregulation von Oberflächenantigenen wie z.B. CD2, CD25 und/oder CD28 und/oder die Sekretion von Cytokinen wie z.B. IL-2 herangezogen werden.

Mit den erfindungsgemäß verwendeten bsAk werden also T-Zellen aktiviert und gegen die Tumorzellen redirigiert. Unspezifische Aktivierungen von T-Zellen hatten in der Immuntherapie generell wenig Erfolg.

Bevorzugte Antikörper sind heterologe bispezifische Antikörper, die aus einer oder mehreren der nachfolgenden Isotypkombinationen ausgewählt werden:

Ratte-IgG2b/Maus-IgG2a,
Ratte-IgG2b/Maus-IgG2b,
Ratte-IgG2b/Maus-IgG3,

Ratte-IgG2b/Human-IgG1,
Ratte-IgG2b/Human-IgG2,
Ratte-IgG2b/Human-IgG3[orientaler Allotyp G3m(st) = Bindung an Protein A],
Ratte-IgG2b/Human-IgG4,

Ratte-IgG2b/Ratte-IgG2c,

Maus-IgG2a/Human-IgG3[kaukasische Allotypen G3m(b+g) = keine Bindung an Protein A, im folgenden mit * gekennzeichnet]

Maus-IgG2a/Maus-[VH-CH1,VL-CL]-Human-IgG1-[Hinge]-Human-IgG3*-[CH2-CH3]

Maus-IgG2a/Ratte-[VH-CH1,VL-CL]-Human-IgG1-[Hinge]-Human-IgG3*-[CH2-CH3]

Maus-IgG2a/Human-[VH-CH1,VL-CL]-Human-IgG1-[Hinge]-Human-IgG3*-[CH2-CH3]

Maus-[VH-CH1,VL-CL]-Human-IgG1/Ratte-[VH-CH1,VL-CL]-Human-IgG1-[Hinge]-Human-IgG3*-[CH2-CH3]

Maus-[VH-CH1,VL-CL]-Human-IgG4/Ratte-[VH-CH1,VL-CL]-Human-IgG4-[Hinge]-Human-IgG4[N-terminale Region von CH2]-Human- IgG3*[C-terminale Region von CH2: > AS-Position 251]-Human- IgG3*[CH3]

Ratte-IgG2b/Maus-[VH-CH1,VL-CL]-Human-IgG1-[Hinge-CH2-CH3]

Ratte-IgG2b/Maus-[VH-CH1,VL-CL]-Human-IgG2-[Hinge-CH2-CH3]

Ratte-IgG2b/Maus-[VH-CH1,VL-CL]-Human-IgG3-[Hinge-CH2-CH3, orientaler Allotyp]

Ratte-IgG2b/Maus-[VH-CH1,VL-CL]-Human-IgG4-[Hinge-CH2-CH3]

Human-IgG1/Human-[VH-CH1,VL-CL]-Human-IgG1-[Hinge]-Human-IgG3*-[CH2-CH3]

Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Ratte-[VH-CH1,VL-CL]-Human-IgG1-[Hinge]-Human-IgG$_2$[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Ratte-[VH-CH1,VL-CL]-Human-IgG1-[Hinge]-Human-IgG3*-[CH2-CH3]

Human-IgG1/Maus-[VH-CH1,VL-CL]-Human-IgG1-[Hinge]-Human-IgG3*-[CH2-CH3]

Human-IgG2/Human-[VH-CH1,VL-CL]-Human-IgG2-[Hinge]-Human-IgG3*-[CH2-CH3]

Human-IgG4/Human-[VH-CH1,VL-CL]-Human-IgG4-[Hinge]-Human-IgG3*-[CH2-CH3]

Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]

Maus-IgG2b/Ratte-[VH-CH1,VL-CL]-Human-IgG1-[Hinge]-Human-IgG3*-[CH2-CH3]

Maus-IgG2b/Human-[VH-CH1,VL-CL]-Human-IgG1-[Hinge]-Human-IgG3*-[CH2-CH3]

Maus-IgG2b/Maus-[VH-CH1,VL-CL]-Human-IgG1-[Hinge]-Human-IgG3*-[CH2-CH3]

Die beiliegenden Abbildungen dienen zur weiteren Veranschaulichung der Erfindung. Die Abbildungen zeigen:

| Abb.1 | Die Rolle von akzessorischen Zellen bei der Tumor-Immuntherapie mit bispezifischen Antikörpern. |
| Abb.2 | Die graphische Darstellung der Ergebnisse des in vivo-Versuchs. |
| Abb.3 | Beispiel für einen bispezifischen Antikörper der Erfindung. |
| Abb.4 | Trispezifischer F(ab)$_2$-Antikörper |
| Abb.5 | Trispezifischer scFv-Antikörper |

Die erfindungsgemäße Aufgabe zielt darauf ab, durch eine effiziente T-Zell-Antwort gegen Tumorzellen eine Immunität gegen Tumore, insbesondere eine Langzeit-Tumorimmunität, zu induzieren. Dies wird erreicht durch Redirektion von T-Zellen an Tumorzellen mittels intakter bispezifischer Antikörper (bsAk) und gleichzeitiger Bindung von Fc-Rezeptor positiven Zellen an den Fc-Teil des bsAk. Wichtig dabei ist, daß die Fc-Rezeptor positive Zelle durch Bindung von (an der T-Zelle bzw. Tumorzelle) immobilisierten intakten bsAk an den Fc-Rezeptor aktiviert wird. Erfindungsgemäß wird unter "Tumor-Langzeitimmunität" ein Zeitraum verstanden, der sich über zumindest mehrere Jahre erstreckt. Unter "Tumorimmunität" wird erfindungsgemäß die Ausbildung einer humoralen und/oder zellulären Immunität verstanden, die gegen den Tumor des Patienten gerichtet ist.
Es werden aufgrund der erfindungsgemäß vorgeschlagenen Verwendung intakter bispezifischer und/oder trispezifischer Antikörper tumorspezifische Antikörper und/oder tumorspezifische T-Zellen oder weitere Immunzellen ausgebildet, die gegen den Tumor wirksam sind. Die erfindungsgemäß verwendeten bispezifischen oder trispezifischen Antikörper können auch, falls angebracht, in Mischung miteinander verwendet werden.
Die in der vorliegenden Anmeldung beschriebenen bispezifischen und trispezifischen Antikörper weisen insbesondere die in den Patentansprüchen gekennzeichneten Eigenschaften auf, bevorzugt natürlich die im Anspruch 1 in den Merkmalen (a) - (e) gekennzeichneten Eigenschaften. Diese Antikörper können somit zur Induktion einer Tumorimmunität, bevorzugt einer Langzeit-Tumorimmunität, bei Mensch und Tier verwendet werden, wobei hierfür insbesondere die unter (a) - (e) des Anspruchs 1 genannten Eigenschaften verantwortlich sind.

Zur Aktivierung der Fc-Rezeptor positiven Zelle durch den bsAk werden bevorzugt spezifische Subklassen bzw. Subklassenkombinationen des bsAk oder bei trispezifischen Antikörpern ein den Fc-Rezeptor erkennender Bindungsarm eingesetzt. Wie in in vitro Versuchen nachgewiesen werden konnte, sind bspw. bsAk der Subklassenkombination Maus-IgG2a/Ratte-IgG2b in der Lage, an Fc-Rezeptor positive Zellen zu binden und diese gleichzeitig zu aktivieren, was zur Hochregulation bzw. Neuausbildung (Expression) von kostimulatorischen Antigenen wie z.B. CD40, CD80 oder CD86 auf der Zelloberfläche dieser Zellen führt. Dagegen sind bsAk der Subklassenkombination Maus-IgG1/Ratte-IgG2b zwar in der Lage an Fc-Rezeptor positive Zellen zu binden (Haagen et al., Interaction of human monocyte Fcγ receptors with rat IgG2b, J. Immunology., 1995, 154: 1852-1860), können diese aber offenbar nicht in vergleichbarem Maße aktivieren (Gast G.C., Haagen I.-A., van Houten A.A., Klein S., Duits A.J., de Weger R.A., Vroom T.M., Clark M.R., J. Phillips, van Dijk A.J.G., de Lau W.B.M., Bast B.J.E.G. CD8 T-cell activation after intravenous administration of CD3xCD19 bispecific antibody in patients with non-Hodgkin lymphoma. Cancer Immunol. Immunother. 40: 390, 1995).

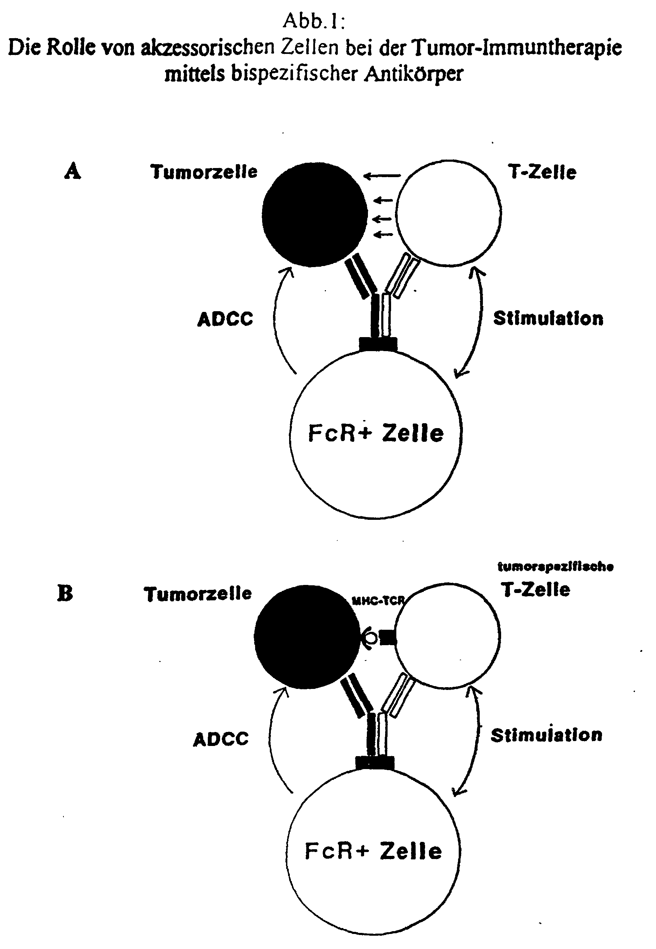

Während der intakte bsAk die T-Zelle mit einem Bindungsarm an CD3 bindet und gleichzeitig aktiviert, können kostimulatorische Signale von der an den Fc-Teil des bsAk gebundenen Fc-Rezeptor positiven Zelle an die T-Zelle übermittelt werden. D.h. erst die Kombination von Aktivierung der T-Zelle über einen Bindungsarm des bsAk und der gleichzeitigen Übertragung von kostimulatorischen Signalen von der Fc-Rezeptor positiven Zelle auf die T-Zelle führt zu einer effizienten T-Zellaktivierung (Abb.1A). Tumorspezifische T-Zellen, die an der Tumorzelle ungenügend aktiviert wurden und deshalb anerg sind, können durch das oben beschriebene erfindungsgemäße Prinzip ebenfalls reaktiviert werden (Abb.1B).

Überraschenderweise konnte somit erfindungsgemäß eine lang andauernde Tumorimmunität durch die erfindungsgemäße Anwendung intakter T-Zell-redirigierender bsAk induziert werden. Diese beruht auf der physiologischen Aktivierung von an den Tumor redirigierten T-Zellen durch 1) Bindung des bsAk an die T-Zelle, beispielsweise über den Zellrezeptor-Komplex, und 2) gleichzeitiger Vermittlung von kostimulatorischen Signalen durch FcR+ Zellen, die an den Fc-Teil des bsAk binden.

Eine wichtige Voraussetzung für eine wirksame Induktion einer Tumorimmunität ist demnach ein bsAk mit einem Fc-Teil, der in der Lage ist, FcR+ Zellen zu binden, die durch dieses Ereignis selbst aktiviert werden und dadurch kostimulatorische Signale an die T-Zelle vermitteln können.

Aufgrund dieses Mechanismus können ebenfalls in Anergie befindliche tumorspezifische T-Zellen (besitzen einen T-Zell-Rezeptor [TCR], welcher tumorspezifische Peptide in Verbindung mit MHC-Molekülen auf der Tumorzelle erkennt) an der Tumorzelle reaktiviert werden und dadurch eine Toleranz gegen den Tumor aufgehoben werden.

Da die tumorspezifischen T-Zellen vollkommen andere Peptide/Proteine über ihren TCR erkennen können als der bsAk mit seinem anti-Tumor-Bindungsarm, können ebenfalls, nach Aktivierung derartiger T-Zellen durch den bsAk, Tumorzellen erkannt und zerstört werden, die von dem bsAk ursprünglich nicht erkannt werden konnten. D.h. der verwendete bsAk muß nicht alle Tumorzellen erkennen, um eine Tumorimmunität zu induzieren, die später alle Tumorzellen erreicht. Aus dieser Erkenntnis folgt weiterhin, daß relativ wenig intakter bsAk (100 μg - 5 mg/Patient) ausreicht, um die erfindungsgemäße Tumorimmunität zu erlangen. Da die Induktion einer derartigen Tumorimmunität eine gewiße Zeit benötigt, sollte der Patient sich, bei Beginn der Therapie, in einer minimal residual disease (MRD) -Situation mit wenigen verbliebenen Tumorzellen befinden. Unter MRD versteht man den Zeitraum nach der Reduktion von größeren Tumormengen mit geeigneten Methoden, wie z.B. der Entfernung des Primärtumors durch chirurgische Maßnahmen, in dem noch residuelle Tumorzellen existieren, die zwar unter Umständen nicht nachweisbar sind, aber nach einer gewissen Zeit zum Rezidiv führen.

Diese hat für die hier beschriebene Therapieform folgende Vorteile:

- Die Anzahl der Tumorzellen und damit das mögliche Auftauchen von sogenannten "Escape-Mutanten", d.h. von Tumorvarianten, welche z.B. ein tumorspezifisches Peptid, das vom Immunsystem erkannt wird, nicht mehr präsentieren, ist stark reduziert.

- Selbst sehr schnell wachsende und aggressive Tumorvarianten haben nach erfolgter "Immunisierung" eine geringere Chance, das Immunsystem quasi zu überrennen.

Die Hauptunterschiede zu einer konventionellen Krebstherapie mit bsAk liegen

1. in der geringen Menge von verabreichtem Antikörper (5 μg - 10 mg, bevorzugt 10 μg - 100 μg, weiterhin 100 μg - 5 mg/Patient); und

2. im Zeitpunkt der Gabe des bsAk, bezogen auf das Krankheitsstadium (bevorzugt in einer MRD-Situation). Es wird bei dieser Form der Therapie nicht abgewartet, bis Tumorzellen, z.B. nach Entfernung eines Primärtumors, wieder nachweisbar sind, sondern es wird prophylaktisch durch Gabe des intakten bsAk eine Art Impfung des Patienten gegen seine Krebszellen durchgeführt;

3. dies ist möglich, da bei derart geringen Mengen von verabreichten intakten bsAk keine schwerwiegenden Nebenwirkungen zu erwarten sind, die den enormen Vorteil - nämlich das Ausbleiben von nicht mehr behandelbaren Metastasen - neutralisieren würden;

4. in der Induktion einer humoralen Immunantwort mit Komplement-fixierenden Antikörpern (beim Menschen die Isoptypen - IgG1, IgG2 und IgG3), die in der Lage sind, Tumorzellen zu zerstören.

Bei den erfindungsgemäß verwendeten Antikörpern handelt es sich vorzugsweise um monoklonale, chimäre, rekombinante, synthetische, halbsynthetische oder chemisch modifizierte intakte Antikörper mit beispielsweise Fv-, Fab-, scFv- oder F(ab)$_2$-Fragmenten.

Sind die erfindungsgemäß verwendeten Antikörper für eine invivo-Therapie vorgesehen, werden bevorzugt Antikörper oder Derivate oder Fragmente vom Menschen verwendet, oder solche, die derart verändert sind, daß sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig. Die Herstellung monoklonaler Antikörper, die ihren Ursprung vorzugsweise in Säugetieren, z.B. Mensch, Ratte, Maus, Kaninchen oder Ziege, haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfré (Meth. Enzymol. 73 (1981), 3) beschrieben sind.

Ferner ist es möglich, die beschriebenen Antikörper mittels rekombinanter DNA-Technologie nach dem Fachmann geläufigen Techniken herzustellen (siehe Kurucz et al., J. Immunol. 154 (1995), 4576; Hollinger et al., Proc. Natl. Acad. Sc. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und rekombinante Zellinien identifiziert und isoliert, die Antikörper mit beiden Spezifitäten produzieren.

Das der Erfindung zugrunde liegende Problem kann sowohl durch bispezifische als auch trispezifische Antikörper gelöst werden, sofern sie die im Anspruch 1 gekennzeichneten Eigenschaften und Wirkungen aufweisen. Nachfolgend wird die Herstellung von Antikörpern mit Zwei- und Dreispezifitäten näher beschrieben. Die Bereitstellung derartiger bispezifischer und trispezifischer Antikörper gehört zum Stand der Technik, und auf die derartige Herstellungstechniken beschreibende Literatur wird hier voll inhaltlich Bezug genommen.

Die Herstellung von Antikörpern mit drei Spezifitäten, sogenannten trispezifischen Antikörpern, durch die das der Erfindung zugrundeliegende Problem ebenfalls lösbar ist, kann beispielsweise derart erfolgen, daß an eine der schweren Ig-Ketten eines bispezifischen Antikörpers eine dritte Antigenbindungsstelle mit einer weiteren Spezifität, z. B. in Form eines "single chain variable fragments" (scFv), angekoppelt wird. Das scFv kann beispielsweise über einen

$$\text{-S-S}(G_4S)_n\text{D-I-Linker}$$

an eine der schweren Ketten gebunden sein (S = Serin, G = Glycin, D = Aspartat, I = Isoleucin).

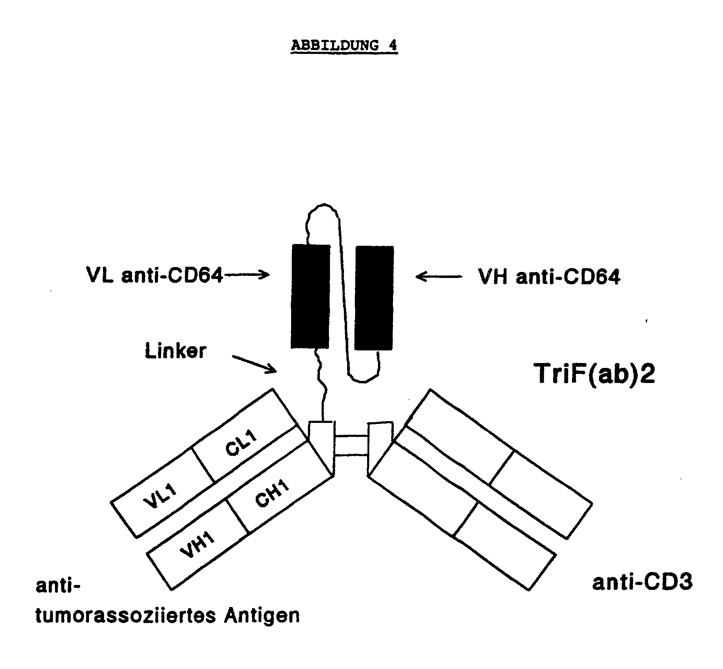

Analog dazu können trispezifische F(ab)$_2$-Konstrukte hergestellt werden, indem die CH2-CH3-Regionen der schweren Kette einer Spezifität eines bispezifischen Antikörpers ersetzt werden durch ein scFv mit einer dritten Spezifität, während die CH2-CH3-Regionen der schweren Kette der anderen Spezifität beispielsweise durch Einbau eines Stopcodons (am Ende der "Hinge"-Region) in das codierende Gen, z.B. mittels homologer Rekombination, entfernt werden (siehe Abb.4).

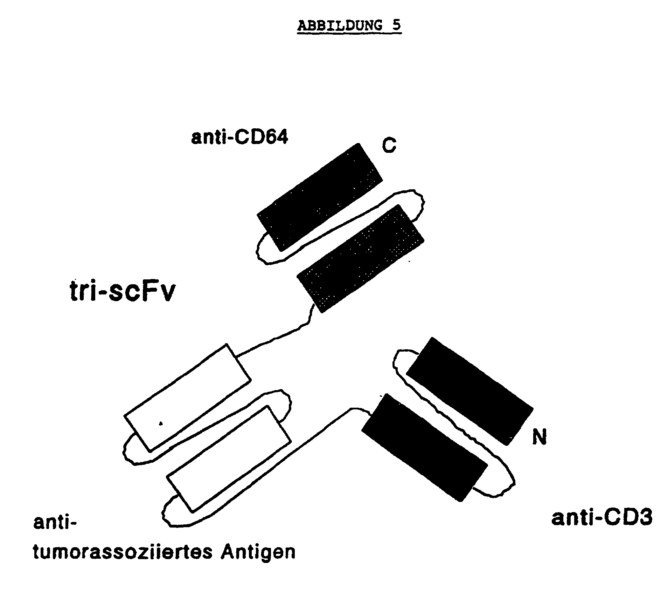

Möglich ist auch die Herstellung trispezifischer scFv-Konstrukte. Hierbei werden drei VH-VL-Regionen, die drei verschiedene Spezifitäten repräsentieren, hintereinander in Reihe angeordnet (Abb.5).

Erfindungsgemäß werden z.B. intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, und besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift, auch bzgl. einer Definition der bispezifischen Antikörper, wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

EP 0 826 696 B1

Beispielsweise können in einem neu entwickelten Herstellungsverfahren (1) intakte bispezifische Antikörper in ausreichender Menge produziert werden. Die Kombination von 2 bispezifischen Antikörpern gegen 2 unterschiedliche tumorassoziierte Antigene (z.B. c-erb-B2, ep-cam, beispielsweise GA-733-2 = C215) auf den Mammakarzinomzellen minimiert die Gefahr, daß Tumorzellen, die nur ein Antigen exprimieren, unerkannt bleiben.

Bispezifische Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Cytokinen oder Apoptose-vermittelnde Mechanismen die Tumorzellen zerstören. Darüber hinaus besteht die Möglichkeit, daß T-Zellen im Rahmen der Aktivierung mit bispezifischen Antikörpern tumorspezifische Antigene über ihren Rezeptor erkennen und dadurch eine dauerhafte Immunisierung eingeleitet wird (Abb.1B). Von besonderer Bedeutung ist dabei der intakte Fc-Teil des bispezifischen Antikörpers, der die Bindung an akzessorische Zellen wie z.B. Monozyten/Makrophagen/Dendriten vermittelt und diese veranlaßt, selbst zytotoxisch zu werden und/ oder gleichzeitig wichtige kostimulatorische Signale an die T-Zelle weiterzugeben (Abb. 1).

Auf diese Weise kann auch eine T-Zellantwort gegen bislang unbekannte, tumorspezifische Peptide induziert werden.

Durch Redirektion von u.U. anergisierten, tumorspezifischen T-Zellen an Tumorzellen mittels bsAk bei gleichzeitiger Kostimulation derartiger T-Zellen durch akzessorische Zellen, welche an den Fc-Teil des bsAk binden, wird die Anergie der CTLs aufgehoben werden. D.h. eine im Patienten gegen den Tumor existierende T-Zell-Toleranz wird erfindungsgemäß mittels intakter bsAk gebrochen und damit eine dauerhafte Tumorimmunität induziert.

Hierfür gibt es erste in vivo-Daten aus Mausversuchen, die auf eine derartige dauerhafte Tumorimmunität nach Behandlung mit einem syngenen Tumor und intakten bsAk hinweisen. In diesen Versuchen überlebten insgesamt 14 von 14 Tieren, die nach einer ersten Tumorinjektion erfolgreich mit bsAk behandelt werden konnten, eine weitere Tumorinjektion 144 Tage nach der ersten - ohne eine erneute Gabe von bsAk (siehe Beispiel 1).

Bei der erfindungsgemäßen Immunisierungsstrategie werden beispielsweise intakte bsAk verwendet, die in der Lage sind, FcγRI+ Zellen über ihren Fc-Teil zu binden. Außerdem besitzen die erfindungsgemäß verwendeten bsAk als zweite Spezifität beispielsweise anti-CD3 neben der tumorzellbindenden Spezifität, d.h. sie sind befähigt, durch den zweiten Bindungsarm an T-Zellen zu binden. Mit den erfindungsgemäß verwendeten bsAk werden also T-Zellen aktiviert und gegen die Tumorzellen redirigiert.

Es wurde auch versucht, durch Behandlung mit bispezifischen F(ab')2-Fragmenten mit den Spezifitäten anti-c-erb-B2 x antiCD64 eine Tumorimmunität zu erreichen. Der Hauptnachteil von bsF(ab')2-Fragmenten liegt darin, daß aufgrund der verwendeten Spezifitäten lediglich FcγRI+ Zellen an den Tumor redirigiert werden. T-Zellen werden durch diesen bispezifischen Antikörper nicht an den Tumor redirigiert. Die bsF(ab')2-Fragmente besitzen zwar auch das Potential zur direkten Tumorzerstörung, sind aber nicht in der Lage, selber eine Tumorimmunität zu etablieren. Dazu ist nur die T-Zelle mit ihrem spezifischen T-Zellrezeptor befähigt. Die FcγRI+ Zellen können zwar indirekt durch Präsentation von tumorspezifischen Peptiden (über MHC I bzw. MHCII) nach z.B. Phagozytose von Tumorzellbestandteilen tumorspezifische T-Zellen aktivieren, die Effizienz der Induktion einer Tumorimmunität ist hier aber nicht ganz so hoch (nur bei 30% der Patienten).

Weitere Vorteile von intakten bsAk mit der Fähigkeit zur Redirektion von T-Zellen gegenüber den o.g. bsF(ab')2 Fragmenten sind im einzelnen:

1. An intakte bsAk können Fc-Rezeptor positive Zellen binden und einerseits über ADCC (antibody-dependent cell-mediated cytotoxicity) direkt zur Tumorzerstörung beitragen sowie andererseits wie oben näher ausgeführt zur T-Zellaktivierung.

2. bsAk mit Fc-Anteil besitzen eine wesentlich längere Zirkulationszeit als z.B. bsF(ab')2 oder bs(scFv) Antikörperfragmente, so daß wesentlich geringere Dosen intakter Ak-Moleküle für eine vergleichbare anti-Tumorwirkung notwendig sind.

3. Durch die erfindungsgemäße Verwendung intakter bispezifischer Antikörper sollen insbesondere residuelle Tumorzellen/Mikrometastasen zerstört werden. Im Gegensatz zur Behandlung solider Tumoren oder größerer Metastasen, bei denen die oben erwähnten Antikörperfragmente Vorteile besitzen könnten, da sie aufgrund ihrer geringeren Größe eine bessere Tumorpenetration ermöglichen.

4. Durch intakte T-Zell-redirigierende bsAk werden auch anergisierte tumorspezifische T-Zellen an die Tumorzelle herangeführt, die erfindungsgemäß direkt am Tumor reaktiviert werden können. Dies kann durch ein bsF(ab')2-Fragment mit den Spezifitäten anti-CD64Xanti-tumorassoziiertes Antigen nicht erreicht werden.

5. Erfindungsgemäßkonnte in Mausversuchen mit T-Zell-redirigierenden intakten bsAk ein Schutz in 100% der Tiere erzielt werden.

Die Bindung des bsAk an Fcγ-RI besitzt zwei wesentliche Vorteile im Hinblick auf eine optimale anti-Tumorwirksamkeit:

Fcγ-RI positive Zellen besitzen die Fähigkeit mittels ADCC Tumorzellen zu eliminieren (2) und können insofern synergistisch zur anti-Tumorwirkung der durch den bsAk an die Tumorzelle herangeführten cytotoxischen T-Zellen beitragen (3).

1. Fcγ-RI positive Zellen (wie z.B. Monozyten/Makrophagen/Dendriten) sind in der Lage, wichtige kostimulatorische Signale, ähnlich wie bei der Antigen-Präsentation, der T-Zelle zu liefern und damit eine Anergisierung der T-Zelle zu verhindern. Wie in Abbildung 1 gezeigt können weiterhin, als erwünschtes Nebenprodukt, aufgrund der durch intakten bsAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monocyt) geliefert werden. Insofern sollte der erfindungsgemäßen Lösung neben der direkten T-Zellrezeptor-unabhängigien durch bsAk vermittelten Tumorzerstörung (Abb.1A), ebenfalls tumorspezifische T-Zellen aktivieren und generieren (Abb.1B), die nach Abbau der bsAk weiterhin im Patienten patrouillieren. D.h. mittels intakter bsAk kann ähnlich wie bei gentherapeutischen Ansätzen (z.B. durch Einbau von kostimulatorischen Antigenen wie B-7 in die Tumorzelle) die Tumortoleranz im Patienten durchbrochen werden.

Günstig in diesem Zusammenhang ist weiterhin, daß die Expression von Fcγ-RI nach G-CSF -Behandlung auf den entsprechenden Zellen hochreguliert wird.

**Beispiel**

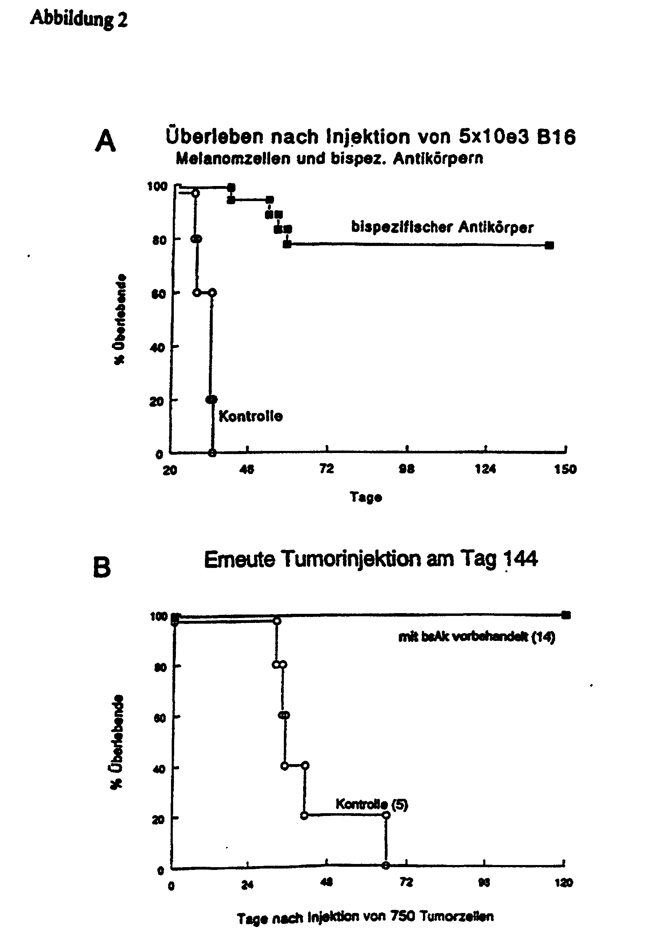

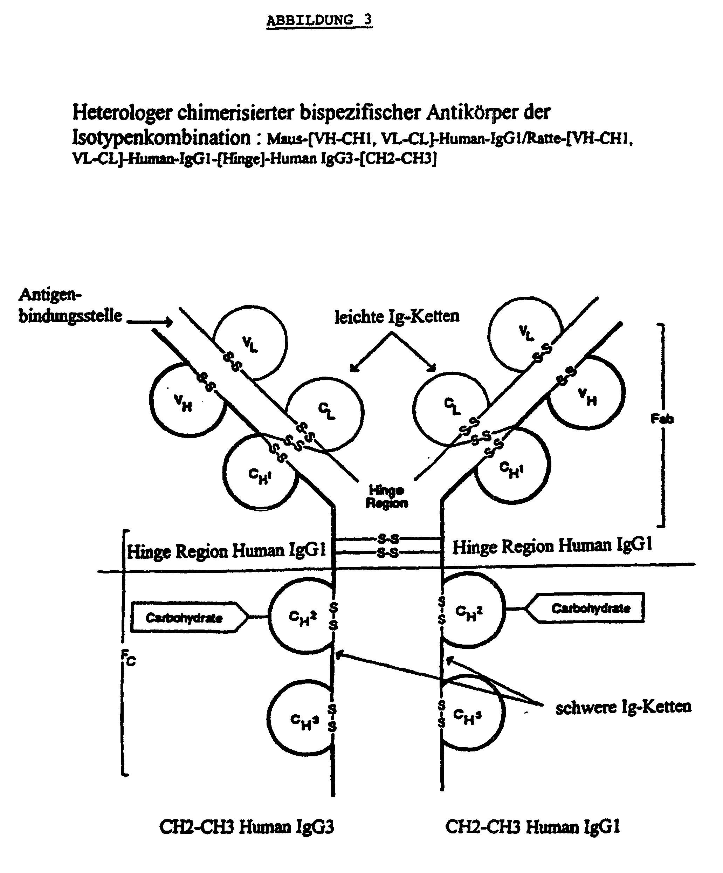

Zur Prüfung der Frage, ob bispezifische Antikörper eine Tumorimmunität induzieren können, wurden C57BL/6 Mäusen zunächst $5x10^3$ syngene B16 Tumorzellen injiziert. Zwei Tage später wurde eine Gruppe von Mäusen (Anzahl 18) mit einem mittels Quadrom-Technologie (1) hergestellten intakten bsAk behandelt, welcher eine Zielstruktur epCAM (C215 = tumorassoziiertes Antigen) auf der Tumorzelle sowie CD3 auf T-Zellen erkennt. Dieser bsAK hat die Subklassenkombination Maus IgG2a/Ratte IgG2b. Eine zweite Gruppe (Anzahl 5) erhielt lediglich eine äquimolare Menge von Fab-Fragmenten der beiden im bsAk enthaltenen Spezifitäten. Während alle Tiere der Fab -Kontrollgruppe innerhalb von 56 Tagen verstarben oder eingeschläfert werden mußten, überlebten 14 der 18 mit bsAk behandelten Tiere. 144 Tage nach der ersten Injektion von Tumorzellen wurde den überlebenden 14 Tieren erneut eine Dosis von 750 B16 Tumorzellen, diesmal ohne Gabe von bsAk, i.p. injiziert. Zur Kontrolle wurde 5 unbehandelten Tieren dieselbe Tumorzellzahl verabreicht. Während das letzte Tier der unbehandelten Kontrollgruppe 66 Tage nach Tumorinjektion eingeschläfert werden mußte, überlebten alle mit bsAk behandelten Tiere (Überwachungszeitraum: 120 Tage nach zweiter Tumorzell-Injektion). Siehe auch Abbildung 2A und B : Überlebenskurven der beiden aufeinanderfolgenden, oben beschriebenen, Experimente.

Diese Versuche zeigen, daß es möglich ist, mit den erfindungsgemäß bereitgestellten bispezifischen Antikörpern, aber auch mit den trispezifischen Antikörpern, eine lang andauernde Tumorimmunität zu induzieren. Es ist davon auszugehen, daß diese Ergebnisse auch auf den Menschen zu übertragen sind, wobei hier von einer Tumor-Langzeitimmunität zumindest über mehrere Jahre hinweg auszugehen ist.

**Nachweis der Induktion einer humoralen Immunantwort mittels intakter bsAk und deren prognostische Bedeutung für das Überleben von Patienten oder Tieren**

**Beschreibung:**

Neben der zellulären Immunität, die hauptsächlich von den T-Zellen getragen wird, spielt die humorale Immunität bei der Tumor-Erkennung und -Zerstörung eine ebenso wichtige Rolle. Dies belegen eine Reihe von Untersuchungen, beispielsweise eine Arbeit von Rodolfo et al. (J.Immunol. 157, 5536, 1996). Rodolfo et al. konnten zeigen, daß (1) durch die Gabe von IL-12 bzw. IL-2 transduzierter Tumorzell-Vakzine tumorspezifische T-Zellen induziert werden; (2) eine effektive humorale Immunantwort mit Komplement-fixierenden Antikörpern nur mit den IL-12 transduzierten Tumorzellen erzeugt werden konnte. Interessanterweise überlebten in diesen Versuchen nur Tiere, die Komplement-fixierende Antikörper mit anti-Tumorspezifität entwikkelt hatten.

In erfindungsgemäß durchgeführten in vitro-Versuchen konnte gezeigt werden, daß mittels der hier beschriebenen intakten bispezifischen Antikörper aufgrund der Bindung und Aktivierung von Fcγ-Rezeptor positiven Zellen (siehe Abb. 1) von diesen Cytokine, beispielsweise IL-12, gebildet und sezerniert werden (siehe Anhang Tabelle 1). Insofern konnte mit den hier beschriebenen intakten bsAk ohne Gentransfer- die Produktion von Cytokinen induziert werden, die ansonsten nur mit Gentransfer im Rahmen von gentherapeutischen Ansätzen zur Expression gebracht werden können. Die Beobachtung ist deshalb so wichtig, weil IL-12 offensichtlich eine wichtige Rolle bei der Induktion einer anti-Tumor-Immunität spielt.

In den im obigen Bsp. 1 gezeigten Tierversuchen konnten in den überlebenden Mäusen, nach bsAk-Behandlung, am Tag 143 nach Tumor-Gabe im Schwanzblut tumorreaktive Komplement-fixierende Antikörper nachgewiesen werden. Dies zeigt, daß für das Überleben der Tiere derartige Antikörper zumindest mit verantwortlich waren, da in verstorbenen Tieren keine oder nur geringe Mengen dieser Antikörper nachgewiesen werden konnten (Tab. 2).

Die vorstehend beschriebenen Versuche wurden anhand bispezifischer Antikörper durchgeführt. Anstelle der bispezifischen können aber auch die in der Anmeldung beschriebenen trispezifischen Antikörper mit den gleichen Ergebnissen eingesetzt werden.

Wie beschrieben werden durch die Behandlung mit den erfindungsgemäß bereitgestellten bispezifischen und trispezifischen Antikörpern tumorreaktive Komplement-bindende Antikörper generiert, die für die Induktion einer humoralen Immunantwort stehen. Dies und die Anregung der Produktion von Cytokinen wie IL-12, dürfte für die erfindungsgemäße Induktion einer Langzeit-Tumorimmunität zumindest mit verantwortlich sein. Es ist deshalb auch möglich, die Menge an Komplement-bindenden tumorreaktiven Antikörpern, die durch Behandlung mit bispezifischen oder trispezifischen Antikörpern gemäß der Erfindung induziert wurden, zur Beurteilung des Krankheitsverlaufs (Prognostik) eines Tumorpatienten zu verwenden. Je höher die Menge an tumorreaktiven Komplement-fixierenden Antikörpern ist, desto günstiger die Prognostik des Tumorpatienten.

Die erfindungsgemäß offenbarte Verwendung z.B. intakter bispezifischer Antikörper erfolgt nach der Behandlung eines Primärtumors, bevorzugt bei Patienten in einer minimal residual disease (MRD)-Situation. Nur bei Patienten mit wenigen verbliebenen Tumorzellen, bei denen allerdings die Gefahr eines Rezidivs hoch ist, wird die erfindungsgemäße Verwendung intakter bispezifischer und /oder trispezifischer Antikörper Bedeutung erlangen.

Literatur

1. Lindhofer et al, Preferential species-restricted heavy-light chain pairing in rat-mouse quadromas: Implications for a single step purification of bispecific antibodies, *J. Immunology 1995,* 155:219

2. Valerius et al., Involvement of the high-affinity receptor for IgG (Fc$\gamma$RI; CD64) in enhanced tumor cell cytotoxicity of neutrophils during granulocyte colonystimulating factor therapy, *Blood, 1993,* 82:931-939

3. Weiner et al.,The role of T cell activation in anti-CD3 x antitumor bispecific antibody therapy, *J. Immunology*, *1994*, 152:2385

Tabelle 1:

| Anstieg von Oberflächenantigenen auf *CD14+ Zellen* (Makrophagen, Monozyten) bzw. Sekretion von Cytokinen nach in vitro Inkubation von PBMC mit intakten bsAk und Tumorzellen nach 20h | | |
| --- | --- | --- |
| Antigen | ohne Ak | mit bsAk |
| CD40 | + | ++ |
| CD80 (B-7.1) | + | ++ |
| CD86 (B-7.2) | + | ++ |
| CD25 (IL-2R) | + | +++ |
| MHC II | + | +(+) |
| bsAk | - | + |
| Mit PCR nachgewiesene Cytokine : IL-1, IL-2, IL-4, IL-6, IL-12 (nur mit bsAk); TNF-$\alpha$ (sowohl mit bsAk als auch mit der Kombination der parentalen Antikörper) | | |

Tabelle 2:

| B16-Melanom Rechallenge Versuch (siehe Bsp.1). Testung der Seren (aus Schwanzblut) von überlebenden Tieren (1-13) sowie am Tumor verstorbener Tiere (14-17) auf tumorreaktive Antikörper. | | | | | |
|---|---|---|---|---|---|
| Maus | Reaktivität mit B16-C215 Zellen in % (Serum Tag 0) | Reaktivität mit B16-C215 Zellen in % (Serum am Tag des Einschläferns bzw. Todes ) | Reaktivität mit B16-C215 Zellen in % (Serum Tag 143) | Komplementfixierende Subklasse mIgG2a | nicht Kompfix. Subklasse mIgG1 |
| 1 | 5 | | 87[a] | +[b] | _[b] |
| 2 | 6 | | 42 | + | - |
| 3 | 5 | | 70 | + | - |
| 4 | 4 | | 73 | + | - |
| 5 | 5 | | 30 | + | - |
| 6 | 5 | | 66 | + | - |
| 7 | 5 | | 57 | + | - |
| 8 | 6 | | 71 | + | - |
| 9 | 5 | | 47 | + | - |
| 10 | 5 | | 49 | + | - |
| 11 | 6 | | 69 | + | - |
| 12 | 5 | | 40 | + | - |
| 13 | 5 | | 54 | + | - |
| 14 | 4 | 8 | | - | - |
| 15 | 5 | 14 | | - | - |
| 16 | 6 | 10 | | - | - |
| 17 | 5 | 18 | | + | - |
| Kontrollserum | 3 | 4 | 2 | - | - |

a) Die Meßungen wurden im FACScan (Becton-Dickinson) durchgeführt. Die tumorgebundenen Antikörper wurden mit polyklonalem fluoreszenzmarkiertem Ratte anti-Maus Antikörper nachgewiesen. Der Zahlenwert ist ein Maß für die Fluoreszenzintensität

b) Untersuchung der tumorreaktiven Mausseren wie unter a) aber mit Isotyp-spezifischen Nachweisantikörpern gerichtet gegen mIgG2a (Komplement-fixierend) bzw. mIgG1 (nicht Komplement-fixierend)

**Patentansprüche**

1. Verwendung eines intakten bispezifischen oder trispezifischen Antikörpers mit den nachfolgenden Eigenschaften und Wirkungen:

(a) bindet über CD3 an eine T-Zelle und vermittelt ihr ein 1. Aktivierungssignal;

(b) bindet an tumorassoziierte Antigene auf einer Tumorzelle;

(c) bindet durch seinen Fc-Teil (bei bispezifischen Antikörpern) oder eine dritte Spezifität (bei trispezifischen Antikörpern) an den Fc-Rezeptor von Fc-Rezeptor positiven Zellen;

(d) aktiviert die Fc-Rezeptor positive Zelle durch seine Bindung an die Fc-Rezeptor positive Zelle, wodurch die Expression von Cytokinen und/oder von kostimulatorischen Antigenen initiiert oder erhöht wird;

(e) die kostimulatorischen Antigene und/oder Cytokine übertragen an die T-Zelle zumindest ein 2. Aktivierungssignal, das für eine physiologische Aktivierung der T-Zelle benötigt wird, wobei sich diese Aktivierung in der Hochregulation von Aktivierungsmarkern, der Zerstörung der Tumorzelle und/oder in einer Proliferation der T-Zelle zeigt;

zur Herstellung eines Arzneimittels zur Induktion einer zumindest gegen die unter b) genannte Tumorzelle gewichteten Tumorimmunität bei Mensch und Tier.

2. Verwendung nach Anspruch 1,
**dadurch gekennzeichnet,**
**daß** der Antikörper weiterhin zur Reaktivierung von in Anergie befindlichen, tumorspezifischen T-Zellen befähigt ist.

3. Verwendung nach Anspruch 1 oder 2,
**dadurch gekennzeichnet,**
**daß** der Antikörper weiterhin befähigt ist, die tumorspezifischen T-Zellen, die über den T-Zell-Rezeptor ein tumorspezifisches Peptid, welches über MHC Klasse I und/oder Klasse II auf Tumorzellen präsentiert wird, erkennen, nach Bindung durch den bispezifischen oder trispezifischen Antikörper wie unter (e) beschrieben ebenfalls zu aktivieren.

4. Verwendung nach Anspruch 1, 2 oder 3,
**dadurch gekennzeichnet,**
**daß** der Antikörper zur Induktion von tumorreaktiven Komplement-bindenden Antikörpern und damit zur Induktion einer humoralen Immunantwort befähigt ist.

5. Verwendung nach einem oder mehreren der vorhergehenden Ansprüche,
**dadurch gekennzeichnet,**
**daß** der Antikörper zur Bindung an Fc-Rezeptor positive Zellen befähigt ist, die einen Fcy-Rezeptor I, II oder III aufweisen.

6. Verwendung nach Anspruch 5,
**dadurch gekennzeichnet,**
**daß** der Antikörper zur Bindung an Monozyten, Makrophagen und/oder Dendriten als Fcy-Rezeptor I positive Zellen befähigt ist.

7. Verwendung nach einem oder mehreren der vorhergehenden Ansprüche,
**dadurch gekennzeichnet,**
**daß** die Expression von CD40, CD80, CD86, ICAM-1 und/oder LFA-3 als kostimulatorische Antigene oder/und die Sekretion von Cytokinen durch die Fc-Rezeptor positive Zelle initiiert oder erhöht wird.

8. Verwendung nach Anspruch 7,
**dadurch gekennzeichnet,**
**daß** die Zytokine IL-1, IL-2, IL 4, IL 6, IL-8, IL-12 und/oder TNF-$\alpha$ sind.

9. Verwendung nach einem oder mehreren der vorhergehenden Ansprüche,
**dadurch gekennzeichnet,**
**daß** die Bindung an die T-Zelle über den T-Zell-Rezeptor-Komplex der T-Zelle erfolgt.

10. Verwendung nach einem oder mehreren der vorhergehenden Ansprüche,
**dadurch gekennzeichnet,**
**daß** der bispezifische Antikörper ein anti-CD3 X anti-Tumor-assoziiertes Antigen-Antikörper ist.

11. Verwendung nach einem oder mehreren der vorhergehenden Ansprüche,
**dadurch gekennzeichnet,**
**daß** der bispezifische Antikörper ein heterologer Ratte/Maus bispezifischer Antikörper ist.

12. Verwendung nach einem oder mehreren der vorhergehenden Ansprüche,
**dadurch gekennzeichnet,**
**daß** der bispezifische Antikörper ein heterologer bispezifischer Antikörper ist, ausgewählt aus einer oder mehreren der nachfolgenden Isotypkombinationen:

Ratte-IgG2b/Maus-IgG2a,
Ratte-IgG2b/Maus-IgG2b,
Ratte-IgG2b/Maus-IgG3,

Ratte-IgG2b/Human-IgG1,
Ratte-IgG2b/Human-IgG2,
Ratte-IgG2b/Human-IgG3[orientaler Allotyp G3m(st) = Bindung an Protein A],
Ratte-IgG2b/Human-IgG4,

Ratte-IgG2b/Ratte-IgG2c,

Maus-IgG2a/Human-IgG3[kaukasische Allotypen G3m(b+g) = keine Bindung an Protein A, im folgenden mit * gekennzeichnet]

Maus-IgG2a/Maus-[VH-CH1,VL-CL]-Human-IgG1-[Hinge]-Human-IgG3*-[CH2-CH3]

Maus-IgG2a/Ratte-[VH-CH1,VL-CL]-Human-IgG1-[Hinge]-Human-IgG3*-[CH2-CH3]

Maus-IgG2a/Human-[VH-CH1,VL-CL]-Human-IgG1-[Hinge]-Human-IgG3*-[CH2-CH3]

Maus-[VH-CH1,VL-CL]-Human-IgGl/Ratte-[VH-CH1,VL-CL]-Human-IgG1-[Hinge]-Human-IgG3*-[CH2-CH3]

Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human- IgG3*[CH3]

Ratte-IgG2b/Maus-[VH-CH1,VL-CL]-Human-IgG1-[Hinge-CH2-CH3]

Ratte-IgG2b/Maus-[VH-CH1,VL-CL]-Human-IgG2-[Hinge-CH2-CH3]

Ratte-IgG2b/Maus-[VH-CH1,VL-CL]-Human-IgG3-[Hinge-CH2-CH3, orientaler Allotyp]

Ratte-IgG2b/Maus-[VH-CH1,VL-CL]-Human-IgG4-[Hinge-CH2-CH3]

Human-IgG1/Human-[VH-CH1,VL-CL]-Human-IgG1-[Hinge]-Human-IgG3*-[CH2-CH3]

Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Ratte-[VH-CH1,VL-CL)-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Ratte-[VH-CH1,VL-CL)-Human-IgG1-[Hinge]-Human-IgG3*-[CH2-CH3]

Human-IgG1/Maus-[VH-CH1,VL-CL]-Human-IgG1-[Hinge]-Human-IgG3*-[CH2-CH3]

Human-IgG2/Human-[VH-CH1,VL-CL]-Human-IgG2-[Hinge]-Human-IgG3*-[CH2-CH3]

Human-IgG4/Human-[VH-CH1,VL-CL]-Human-IgG4-[Hinge]-Human-IgG3*-[CH2-CH3]

Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]

Maus-IgG2b/Ratte-[VH-CH1,VL-CL]-Human-IgG1-[Hinge]-Human-IgG3*-[CH2-CH3]

Maus-IgG2b/Human-[VH-CH1,VL-CL)-Human-IgG1-[Hinge]-Human-IgG3*-[CH2-CH3]

Maus-IgG2b/Maus-[VH-CH1,VL-CL)-Human-IgG1-[Hinge]-Human-IgG3*-[CH2-CH3]

**13.** Verwendung nach einem oder mehreren der vorhergehenden Ansprüche,
**dadurch gekennzeichnet,**
**daß** der trispezifische Antikörper ein anti-CD3 X anti-Tumor-assoziiertes Antigen-Antikörper mit einem zusätzlichen anti-Fc-Rezeptor-Bindungsarm ist.

**14.** Verwendung eines Antikörpers nach einem oder mehreren der vorhergehenden Ansprüche zur Herstellung eines Arzneimittels zur Applikation am Patienten in einer Menge von 5µg-10 mg/Patient in einer minimal residual disease (MRD)-Situation.

**Claims**

**1.** Use of an intact bispecific or trispecific antibody having the following properties and effects of:

(a) binding to a T cell via CD3 and mediating a first activation signal thereto;
(b) binding to tumour-specific antigens on a tumour cell;
(c) binding, through its Fc portion (in the case of bispecific antibodies) or a third specificity (in the case of trispecific antibodies) to the Fc receptor of Fc receptor-positive cells;
(d) activating of the Fc receptor-positive cell by binding to the Fc receptor-positive cell and, thereby initiating or increasing the expression of cytokines and/or co-stimulatory antigens;
(e) transfer of at least a second activation signal required for pysiological activation of the T cell to the T cell by the co-stimulatory antigens and/or cytokines, this activation being indicated by up-regulation of activation markers, killing of the tumour cell, and/or T cell proliferation;

for the preparation of a medicament for the induction of an anti-tumour immunity which is directed at least against the tumour cell indicated in step b) in men and animals.

**2.** Use according to claim 1,
**characterized in that**
said antibody further is able to reactivate tumour-specific T calls being in a state of anergy.

**3.** Use according to claim 1 or 2,
**characterized in that**
said antibody further is able to activate the tumour-specific T cells recognizing a tumour-specific peptide presented on the tumour cells by MHC class I and/or class II via their T cell receptor upon binding to the bispecific or trispecific antibody as described under (e).

**4.** Use according to claim 1, 2 or 3,
**characterized in that**
said antibody is able to induce tumour-reactive complement-binding antibodies and, thereby, to induce a humoral immune reaction.

**5.** Use according to one or more of the previous claims,
**characterized in that**
said antibody is able to bind to Fc receptor-positive cells having a Fcγ receptor I, II, or III.

**6.** Use according to claim 5,
**characterized in that**
said antibody is able to bind to monocytes, makrophages and/or dendritic cells being Fcγ receptor I-positive cells.

**7.** Use according to one or more of the previous claims,
**characterized in that**
by the Fc receptor-positive cell the expression of the co-stimulatory antigens CD40, CD80, CD86, ICAM-1, and/or LFA-3 and/or the secretion of cytokines is initiated or increased.

**8.** Method according to claim 7,

**characterized in that**
the cytokines are IL-1, IL-2, IL-4, IL-6, IL-8, IL-12, and/or TNF-α.

9. Use according to one or more of the previous claims,
**characterized in that**
said binding to the T cell takes place via the T cell receptor complex of the T cell.

10. Use according to one or more of the previous claims,
**characterized in that**
said bispecific antibody is an anti-CD3 X anti-tumour-associated antigen antibody.

11. Use according to one or more of the previous claims,
**characterized in that**
said bispecific antibody is a heterologous rat/mouse bispecific antibody.

12. Use according to one or more of the previous claims,
**characterized in that**
said bispecific antibody is a heterologous bispecific antibody selected of one or more of the following combinations of isotypes:

rat-IgG2b/mouse-IgG2a,
rat-IgG2b/mouse-IgG2b,
rat-IgG2b/mouse-IgG3,

rat-IgG2b/human-IgG1,
rat-IgG2b/human-IgG2,
rat-IgG2b/human-IgG3 [oriental allotype G3m(st) binding to protein A],
rat-IgG2b/human-IgG4,

rat-IgG2b/rat-IgG2c,

mouse-IgG2a/human-IgG3[caucasian allotypes G3m(b+g) no binding to protein A, in the following indicated as *]

mouse-IgG2a/mouse-[VH-CH1,VL-CL]-human-IgG1-[hinge]-human-IgG3*-[CH2-CH3]

mouse-IgG2a/rat-[VH-CH1,VL-CL]-human-IgG1-[hinge]-human- IgG3*-[CH2-CH3]

mouse-IgG2a/human-[VH-CH1,VL-CL]-human-IgG1-[hinge]-human-LgG3*-[CH2-CH3]

mouse-[VH-CH1,VL-CL]-human-IgG1/rat-[VH-CH1,VL-CL]-human-IgG1-[hinge]-human-IgG3*-[CH2-CH3]
mouse-[VH-CH1,VL-CL]-human-IgG4/rat-[VH-CH1,VL-CL]-human-IgG4-[hinge]-human-IgG4[N-terminal region of CH2]-human-IgG3*[C-terminal region of CH2: > aa position 251]-human-IgG3*[CH3]

rat-IgG2b/mouse-[VH-CH1,VL-CL]-human-IgG1-[hinge-CH2-CH3]

rat-IgG2b/mouse-[VH-CH1,VL-CL]-human-IgG2-[hinge-CH2-CH3]

rat-IgG2b/mouse-[VH-CH1,VL-CL]-human-IgG3-[hinge-CH2-CH3,     oriental     allotype]     rat-IgG2b/
mouse-[VH-CH1,VL-CL]-human-IgG4-[hinge-CH2-CH3]human-IgG1/human-[VH-CH1,VL-CL]-human-
IgG1-[hinge]-human-IgG3*-[CH2-CH3]

human-IgG1/rat-[VH-CH1,VL-CL]-human-IgG1-[hinge]-human-IgG4[N-terminal region of CH2]-human-IgG3*
[C-terminal region of CH2 : > aa position 251]-human-IgG3*[CH3]

human-IgG1/mouse-[VH-CH1,VL-CL]-human-IgG1-[hinge]-human-IgG4[N-terminal  region  of  CH2]-human-
IgG3*[C-terminal region of CH2 : > aa position 251]-human-IgG3*[CH3]

human-IgG1/rat-[VH-CH1,VL-CL]-human-IgG1-[hinge]-human-IgG2[N-terminal region of CH2]-human-IgG3*[C-terminal region of CH2 : > aa position 251]-human-IgG3*[CH3]

human-IgG1/mouse-[VH-CH1,VL-CL]-human-IgG1-[hinge]-human-IgG2[N-terminal region of CH2]-human-IgG3*[C-terminal region of CH2: > aa position 251]-human-IgG3*[CH3]

human-IgG1/rat-[VH-CH1,VL-CL]-human-IgG1-[hinge]-human-IgG3*-[CH2-CH3]

human-IgG1/mouse-[VH-CH1,VL-CL]-human-IgG1-[hinge]-human-IgG3*-[CH2-CH3]

human-IgG2/human-[VH-CH1,VL-CL]-human-IgG2-[hinge]-human-IgG3*-[CH2-CH3]

human-IgG4/human-[VH-CH1,VL-CL]-human-IgG4-[hinge]-human-IgG3*-[CH2-CH3]

human-IgG4/human-[VH-CH1,VL-CL]-human-IgG4-[hinge]-human-IgG4[N-terminal region of CH2]-human-IgG3*[C-terminal region of CH2 : > aa position 251]-human-IgG3*[CH3]

mouse-IgG2b/rat-[VH-CH1,VL-CL]-human-IgG1-[hinge]-human-IgG3*-[CH2-CH3]

mouse-IgG2b/human-[VH-CH1,VL-CL]-human-IgG1-[hinge]-human-IgG3*-[CH2-CH3]

mouse-IgG2b/mouse-[VH-CH1,VL-CL]-human-IgG1-[hinge]-human-IgG3*-[CH2-CH3].

13. Use according to one or more of the previous claims,
**characterized in that**
said trispecific antibody is an anti-CD3 X anti-tumour-associated antigen antibody having an additional anti-Fc binding arm.

14. Use of an antibody according to one or more of the previous claims for the preparation of a medicament administered in an amount of 5 μg - 10 mg/patient by application to the patient being in a minimal residual disease (MRD) situation.


**Revendications**

1. Utilisation d'un anticorps bispécifique ou trispécifique intact ayant les propriétés et actions suivantes :

(a) se fixe par CD3 à un lymphocyte T et lui transmet un premier signal d'activation ;
(b) se fixe à des antigènes associés à des tumeurs sur une cellule tumorale ;
(c) se fixe par son fragment Fc (dans le cas d'anticorps spécifiques) ou par une troisième spécificité (dans le cas d'anticorps trispécifiques) au récepteur Fc de cellules récepteur Fc-positives ;
(d) active la cellule récepteur Fc-positive par sa fixation à la cellule récepteur Fc-positive, ce par quoi l'expression de cytokines et/ou d'antigènes costimulateurs est déclenchée ou augmentée ;
(e) les cytokines et/ou les antigènes costimulateurs transmettent au lymphocyte T au moins un deuxième signal d'activation qui est requis pour une activation physiologique du lymphocyte T, cette activation se manifestant par la stimulation de marqueurs d'activation, la destruction de la cellule tumorale et/ou par une prolifération des lymphocytes T ;

pour la fabrication d'un médicament destiné à l'induction d'une immunité tumorale dirigée au moins contre la cellule tumorale mentionnée en b), chez l'homme et l'animal.

2. Utilisation selon la revendication 1, **caractérisée en ce que** l'anticorps est en outre apte à la réactivation de lymphocytes T spécifiques de tumeurs, se trouvant en anergie.

3. Utilisation selon la revendication 1 ou 2, **caractérisée en ce que** l'anticorps est en outre capable d'activer également les lymphocytes T, spécifiques de tumeurs, qui reconnaissent par l'intermédiaire du récepteur de lymphocyte T un peptide spécifique de tumeur qui est présenté sur des cellules tumorales par le CMH de classe I et/ou, de classe II, après fixation par l'anticorps bispécifique ou trispécifique comme décrit en (e).

**4.** Utilisation selon la revendication 1, 2 ou 3, **caractérisée en ce que** l'anticorps est apte à l'induction d'anticorps réactifs avec des tumeurs, se fixant au complément, et par conséquent à l'induction d'une réponse immunitaire humorale.

**5.** Utilisation selon une ou plusieurs des revendications précédentes, **caractérisée en ce que** l'anticorps est capable de se fixer aux cellules récepteur Fc-positives qui présentent un récepteur Fc$\gamma$ I, II ou III.

**6.** Utilisation selon la revendication 5, **caractérisée en ce que** l'anticorps est capable de se fixer à des monocytes, macrophages et/ou dendrites, en tant que cellules récepteur Fc$\gamma$ I-positives.

**7.** Utilisation selon une ou plusieurs des revendications précédentes, **caractérisée en ce que** l'expression de CD40, CD80, CD86, ICAM-1 et/ou LFA-3 en tant qu'antigènes costimulateurs ou/et la sécrétion de cytokines par les cellules récepteur Fc-positives est(sont) déclenchée(s) ou accrue(s).

**8.** Utilisation selon la revendication 7, **caractérisée en ce que** les cytokines sont IL-1, IL-2, IL-4, IL-6, IL-8, IL-12 et/ou TNF-$\alpha$.

**9.** Utilisation selon une ou plusieurs des revendications précédentes, **caractérisée en ce que** la fixation au lymphocyte T s'effectue par l'intermédiaire du complexe des récepteurs de lymphocyte T du lymphocyte T.

**10.** Utilisation selon une ou plusieurs des revendications précédentes, **caractérisée en ce que** l'anticorps bispécifique est un anticorps anti-CD3 $\times$ anti-antigène associé à une tumeur.

**11.** Utilisation selon une ou plusieurs des revendications précédentes, **caractérisée en ce que** l'anticorps bispécifique est un anticorps bispécifique hétérologue rat/souris.

**12.** Utilisation selon une ou plusieurs des revendications précédentes, **caractérisée en ce que** l'anticorps bispécifique est un anticorps bispécifique hétérologue choisi parmi une ou plusieurs des combinaisons d'isotypes suivantes :

IgG2b de rat/IgG2a de souris,
IgG2b de rat/IgG2b de souris,
IgG2b de rat/IgG3 de souris,
IgG2b de rat/IgG1 humaine;
IgG2b de rat/IgG2 humaine,
IgG2b de rat/IgG3 humaine [allotype oriental G3m(st) = liaison à la protéine A],
IgG2b de rat/IgG4 humaine,

IgG2b de rat/IgG2c de rat,

IgG2a de souris/IgG3 humaine [allotype caucasien G3m(b+g) = pas de liaison à la protéine A, **caractérisé par** * ci-après]

IgG2a de souris/[VH-CH1,VL-CL] de souris-IgG1 humaine-[charnière]-IgG3* humaine-[CH2-CH3]

IgG2a de souris/[VH-CH1,VL-CL] de rat-IgG1 humaine-[charnière]-IgG3* humaine-[CH2-CH3]

IgG2a de souris/[VH-CH1,VL-CL] humain-IgG1 humaine-[charnière]-IgG3* humaine-[CH2-CH3]

[VH-CH1,VL-CL] de souris-IgG1 humaine/[VH-CH1,VL-CL] de rat-IgG1 humaine-[charnière]-IgG3* humaine-[CH2-CH3]

[VH-CH1,VL-CL] de souris-IgG4 humaine/[VH-CH1,VL-CL] de rat-IgG4 humaine-[charnière]-IgG4 humaine [région N-terminale de CH2]-IgG3* humaine [région C-terminale de CH2 : > position d'aa 251]-IgG3* humaine [CH3]

IgG2b de rat/[VH-CH1,VL-CL] de souris-IgG1 humaine-[charnière-CH2-CH3]

IgG2b de rat/[VH-CH1,VL-CL] de souris-IgG2 humaine-[charnière-CH2-CH3]

IgG2b de rat/[VH-CH1,VL-CL] de souris-IgG3 humaine-[charnière-CH2-CH3, allotype oriental]

IgG2b de rat/[VH-CH1,VL-CL] de souris-IgG4 humaine-[charnière-CH2-CH3]

IgG1 humaine/[VH-CH1,VL-CL] humain-IgG1 humaine-[charnière]-IgG3* humaine-[CH2-CH3]

IgG1 humaine/[VH-CH1,VL-CL] de rat-IgG1 humaine-[charnière]-IgG4 humaine[région N-terminale de CH2]-IgG3* humaine[région C-terminale de CH2 : > position d'aa 251]-IgG3* humaine[CH3]

IgG1 humaine/[VH-CH1,VL-CL] de souris-IgG1 humaine-[charnière]-IgG4 humaine[région N-terminale de CH2]-IgG3* humaine[région C-terminale de CH2 : > position d'aa 251]-IgG3* humaine[CH3]

IgG1 humaine/[VH-CH1,VL-CL] de rat-IgG1 humaine-[charnière]-IgG2 humaine[région N-terminale de CH2]-IgG3* humaine[région C-terminale de CH2 > position d'aa 251]-IgG3* humaine[CH3]

IgG1 humaine/[VH-CH1,VL-CL] de souris-IgG1 humaine-[charnière]-IgG2 humaine[région N-terminale de CH2]-IgG3* humaine[région C-terminale de CH2 : > position d'aa 251]-IgG3* humaine[CH3]

IgG1 humaine/[VH-CH1,VL-CL] de rat-IgG1 humaine-[charnière]-IgG3* humaine[CH2-CH3]

IgG1 humaine/[VH-CH1,VL-CL] de souris-IgG1 humaine-[charnière]-IgG3* humaine[CH2-CH3]

IgG2 humaine/[VH-CH1,VL-CL] humain-IgG2 humaine-[charnière]-IgG3* humaine[CH2-CH3]

IgG4 humaine/[VH-CH1,VL-CL] humain-IgG4 humaine-[charnière]-IgG3* humaine[CH2-CH3]

IgG4 humaine/[VH-CH1,VL-CL] humain-IgG4 humaine-[charnière]-IgG4 humaine[région N-terminale de CH2]-IgG3* humaine[région C-terminale de CH2 : > position d'aa 251]-IgG3* humaine[CH3]

IgG2b de souris/[VH-CH1,VL-CL] de rat-IgG1 humaine-[charnière]-IgG3* humaine[CH2-CH3]

IgG2b de souris/[VH-CH1,VL-CL] humain-IgG1 humaine-[charnière]-IgG3* humaine[CH2-CH3]

IgG2b de souris/[VH-CH1,VL-CL] de souris-IgG1 humaine-[charnière]-IgG3* humaine[CH2-CH3].

13. Utilisation selon une ou plusieurs des revendications précédentes, **caractérisée en ce que** l'anticorps trispécifique est un anticorps anti-CD3 $\times$ anti-antigène associé à une tumeur, comportant un bras de liaison supplémentaire anti-récepteur Fc.

14. Utilisation d'un anticorps selon une ou plusieurs des revendications précédentes, pour la fabrication d'un médicament destiné à l'administration au patient en une quantité de 5 μg-10 mg/patient, dans un cas de maladie résiduelle imperceptible (MRD = *minimal residual disease)*

Abb.1:
# Die Rolle von akzessorischen Zellen bei der Tumor-Immuntherapie mittels bispezifischer Antikörper

**A**     **Tumorzelle**          **T-Zelle**

**ADCC**          **Stimulation**

**FcR+ Zelle**

**B**     **Tumorzelle**   MHC-TCR   **tumorspezifische T-Zelle**

**ADCC**          **Stimulation**

**FcR+ Zelle**

**Abbildung 2**

**A** **Überleben nach Injektion von 5x10e3 B16 Melanomzellen und bispez. Antikörpern**

**B** **Erneute Tumorinjektion am Tag 144**

ABBILDUNG 3

Heterologer chimerisierter bispezifischer Antikörper der
Isotypenkombination : Maus-[VH-CH1, VL-CL]-Human-IgG1/Ratte-[VH-CH1,
VL-CL]-Human-IgG1-[Hinge]-Human IgG3-[CH2-CH3]

ABBILDUNG 4

VL anti-CD64 ⟶

VH anti-CD64 ⟵

Linker

TriF(ab)2

CL1

VL1

CH1

VH1

anti-CD3

anti-
tumorassoziiertes Antigen

ABBILDUNG 5